# EUROPÄISCHE PATENTSCHRIFT

(11) **EP 0 854 133 B1**
(45) Veröffentlichungstag und Bekanntmachung des Hinweises auf die Patenterteilung: **26.06.2002**
(21) Anmeldenummer: 98100435.1
(22) Anmeldetag: 13.01.1998
(51) Int. Cl.: C07C 225/14, A61K 7/42

(54) **Arylaminomethylencampherderivate**
Arylaminomethylenecamphor derivates
Dérivés d'arylaminomethylenecamphre

(30) Priorität: 17.01.1997 DE 19701448
(43) Veröffentlichungstag der Anmeldung: 22.07.1998
(73) Patentinhaber: BASF AKTIENGESELLSCHAFT, 67056 Ludwigshafen (DE)
(72) Erfinder: Drögemüller, Michael Dr., 68167 Mannheim (DE); Bomm, Volker Dr., 67112 Mutterstadt (DE); Schehlmann, Volker Dr., 67354 Römerberg (DE); Westenfelder, Horst, 67435 Neustadt (DE)

(56) Entgegenhaltungen:
- DE-A- 2 336 219
- SINGH: "Studies on the dependence of optical rotary power on chemical constitution, XVII. Nitro and carboxy-aryl derivatives of stereoisomeric methylenecamphors" J. INDIAN. CHEM. SOC. , Bd. 17, 1940, Seiten 1-18, XP002063069
- RUPE: "Stereoisomere Abkömmlinge des Aminomethylenecamphers" HELVETICA CHIMICA ACTA, Bd. III, 1920, BASEL CH, Seiten 71-89, XP002063070
- CHEMICAL ABSTRACTS, vol. 32, no. 10, 1938 Columbus, Ohio, US; "Dependence of optical rotary power on chemical constitution, XV. Chloroaryl derivatives of stereoisomeric methylenecamphors" Spalte 3763; XP002063072 & PROC. INDIAN ACAD. SCI., Bd. 6A, 1937, Seiten 340-358,
- KNORR, RUDOLF ET AL: "E/Z-Equilibrium. X. Syntheses and tautomerism of.beta.-keto-enamines and vinamidines with the bornene skeleton" CHEM. BER. (1985), 118(11), 4486-95 CODEN: CHBEAM;ISSN: 0009-2940, 1985, XP002063071
- CHEMICAL ABSTRACTS, vol. 74, no. 7, 1971 Columbus, Ohio, US; abstract no. 31852, "Camphor derivatives. I. Compounds of (-)-(hydroxymethylene)camphor with primary amines" XP002063074 & TERENT'EV: ZH, OBSHCH. KHIM., Bd. 40, Nr. 6, 1970, Seiten 1398-404,
- CHEMICAL ABSTRACTS, vol. 14, no. 11, 1920 Columbus, Ohio, US; "Stereoisomerism of derivatives of aminomethylen camphor. I" Seite 1665; XP002063075 & HELVETICA CHIM. ACTA, Bd. 3, 1920, Seiten 50-70,
- SINGH ET AL. : "Studies on the dependence of optical rotary power on chemical constitution, Part XXI. The rotary dispersion of stereoisomeric hydroxyphenylaminomethylenecamphors and their acetylated and benzoylated derivatives" J. IND. CHEM. SOC. , Bd. XX, 1943, Seiten 1-10, XP002063216
- V.N. GOGTE ET AL. : "Tautomerism in 2-arylaminomethylenecamphors" IND. J. CHEM., Bd. 15B, 1977, Seiten 778-781, XP002063217

## Beschreibung

Die vorliegende Erfindung betrifft die Verwendung der Arylaminomethylencampherderivate der Formel (1), 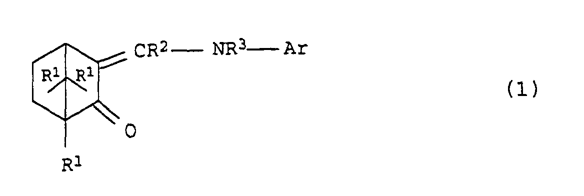 als Lichtschutzmittel, insbesondere in kosmetischen Formulierungen.

Lichtschutzfilter auf der Basis von Campherderivaten sind bekannt. In DE 23 36 219 sind sulfonierte Benzyliden-und Cinnamylidencampherderivate beschrieben, die am Phenylring in der 4-Position unsubstituiert oder durch Methyl, Methoxy oder Chlor substituiert sind.

In DE 34 45 712 werden eine Reihe von ungesättigten Campherderivaten, bevorzugt Benzylidencampherderivate, beschrieben, die sich als Arzneimittel zur Behandlung von Hauterkrankungen eignen.

In DE 44 26 216 werden Benzyliden-Norcampherderivate beschrieben, die als Sonnenfilter und zur Vorbeugung von Entzündungen und Hauterkrankungen einsetzbar sind.

In DE 44 24 489 wird ein Verfahren zur Herstellung von substituierten 4-Methylidenzimtsäurederivaten beschrieben.

Lichtschutzfilter auf der Basis von vinylogen Amiden sind ebenfalls bekannt. In DE 33 16 287 wird eine Reihe von vinylogen Amiden mit offenkettiger Struktur beschrieben, die sich als Lichtfilter in Sonnenschutzmitteln eignen.

Die Anforderungen, die an ein Lichtschutzmittel, das als UV-A-Filter eingesetzt werden soll, gestellt werden, sind zahlreich (Suncreens, ed. N.J. Lowe, N.A. Shaath, Marcel Dekker Inc., New York 1990, S. 230 - 231). Die wichtigsten sind:
1) Es hat sein Absorptionsmaximum im Bereich der UV-A-Region von 320 bis 360 nm;
2) es hat eine hohe spezifische Absorption in diesem Bereich;
3) es ist farblos, d.h. die Absorption oberhalb 400 nm sollte verschwindend gering sein, um eine Einfärbung des Hautschutzpräparates oder der Kleidung nach Anwendung auszuschließen;
4) es ist photo- und thermostabil;
5) es ist hautverträglich und führt nicht zu reizenden oder toxischen Wirkungen gegenüber der Haut;
6) es hat eine gute Haftung auf der Haut;
7) es ist mit kosmetischen Substanzen verträglich und in kosmetischen Lösungsmitteln und Zubereitungen gut löslich;
8) es ist isomerenrein.

Die bekannten Cinnamylidencampherderivate sind Breitband-UV-Filter, die keine ausreichende Schutzwirkung im UV-A-Bereich entfalten. Ferner ist ihre Löslichkeit speziell in der Ölphase für einige Anwendungen nicht befriedigend.

Es bestand daher die Aufgabe, eine als UV-A Filter geeignete Verbindung bereitzustellen, die vor allem eine gute Photostabilität, gute Löslichkeit in der Ölphase und ein ausgeprägtes Absorptionsmaximum im UV-A Bereich aufweist.

Es wurde gefunden, daß Arylaminomethylencampherverbindungen der Formel (1), 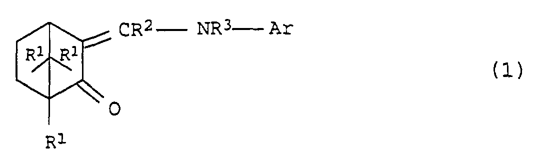 wobei die C=C Doppelbindung in der Z oder E Konfiguration vorliegt, und
- R¹ =: H, CH₃,
- R² =: H, C₁-C₆-Alkyl, C₃-C₈-Cycloalkyl, C₂-C₆-Alkenyl, C₃-C₈-Cycloalkenyl, Aryl oder substituiertes Aryl,
- R³ =: H, C₁-C₆-Alkyl, C₃-C₈-Cycloalkyl, Aryl, substituiertes Aryl, C₁-C₆-Alkoxy, C₁-C₈-Acyl,
- Ar =A: ryl, substituiertes Aryl, Heteroaryl, substituiertes Heteroaryl,
bedeuten, die bestehende Aufgabe in hervorragender Weise lösen.

Die Herstellung von (1) erfolgt, indem Hydroxymethylencampherverbindungen der allgemeinen Formel (2) (hergestellt gemäß L. Claisen, Ann. 281, 1894, S. 306) mit Aminen der allgemeinen Formel (3), in Gegenwart einer Base oder einer Säure sowie gegebenenfalls unter Zusatz von einem oder mehrerer Lösungsmittel, bei

Temperaturen zwischen 0°C und 200°C umgesetzt werden.

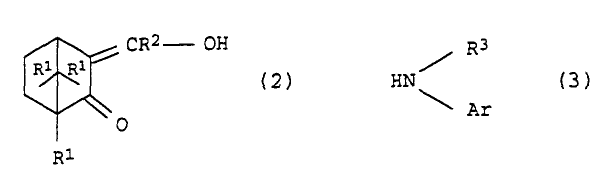

Zur Herstellung der erfindungsgemäßen Verbindungen werden (2) und (3) equimolar umgesetzt.

Die Reaktion wird im allgemeinen in protonischen Lösungsmitteln, z.B. Methanol oder Ethanol durchgeführt. Sie läßt sich aber auch in Ethern, z.B. Diethylether oder Tetrahydrofuran, in Paraffingemischen mit C₁ bis C₂₀ oder in anderen aliphatischen oder aromatischen Lösungsmitteln wie Hexan, Toluol oder Xylol durchführen.

Es ist aber auch möglich, die Reaktion in Substanz, d.h. ohne Lösungsmittel durchzuführen. Bevorzugt wird in niederen Alkoholen gearbeitet und zur Erlangung einer guten Raum-Zeit-Ausbeute in so wenig Lösungsmittel gearbeitet, daß bei der Reaktionstemperatur eine rührbare Mischung entsteht.

Die Reihenfolge, in der (2) und (3) zur Reaktion gebracht werden, ist beliebig. Der Katalysator kann vorgelegt oder auch zudosiert werden. Die Reaktion ist auch als Eintopfreaktion durchführbar.

Ebenso kann (2) zusammen mit dem Katalysator vorgelegt und (3) zudosiert werden und umgekehrt. Auch ist es denkbar, den Katalysator zusammen mit (3) vorzulegen und (2) zuzudosieren.

Die Reaktion kann in einem weiten Temperaturintervall von 0°C bis + 200°C durchgeführt werden. Bevorzugt ist der Temperaturbereich von 20°C bis 100°C. Besonders bevorzugt ist der Temperaturbereich von 60°C bis 90°C.

Die Reaktionszeit hängt unmittelbar von der Reaktionstemperatur ab. Im allgemeinen ist die Reaktion im besonders bevorzugten Temperaturbereich nach 1 bis 5 Stunden beendet. Bei niedrigeren Temperaturen oder geringen Stationärkonzentrationen kann sich die Reaktionszeit beträchtlich verlängern und bis zu 48 h in Anspruch nehmen.

Als Katalysatoren können beliebige organische oder anorganische Basen oder auch Mischungen davon verwendet werden. Typische basische Katalysatoren sind beispielsweise Pyridin, Trimethylamin oder anorganische Carbonate. Die Basen können im Reaktionsgemisch homogen oder auch heterogen vorliegen.

Vorzugsweise werden zur Reaktion organische oder anorganische Säuren oder auch Mischungen davon als Katalysatoren verwendet. Als anorganische Säuren können u.a. Salzsäure, Schwefelsäure, Salpetersäure und/oder Phosphorsäure Verwendung finden.

Als organische Säuren können u.a. Ameisensäure, Essigsäure, Oxalsäure, Bernsteinsäure, Ascorbinsäure und/oder Sulfonsäuren, wie z.B. Methansulfonsäure oder Toluolsulfonsäure eingesetzt werden.

Darüberhinaus sind saure Ionenaustauscher als Katalysatoren für die Umsetzung von (2) mit (3) ebenfalls geeignet.

Die Isolierung des Wertproduktes (1) erfolgt durch die üblichen Techniken, wie Sedimentieren, Filtrieren, Zentrifugieren, Phasentrennung und Extrahieren mit Lösungsmitteln.

Die Reinigung des Wertproduktes (1) kann durch Umkristallisation aus organischen Lösungsmitteln und deren Gemischen und/oder Wasser erfolgen. Bevorzugt sind Mischungen, die Alkohole enthalten.

Die Reinigung kann auch durch Ionenschmelzen und chromatographische Methoden aber auch durch Destillation erfolgen.

Die erfindungsgemäßen Verbindungen (1) können aus einem Campherteil (R¹ = CH₃) oder einem Norcampherteil (R¹ = H) bestehen.

Der Rest R² besitzt die folgende Bedeutung:

H; C₁-C₆-Alkyl, bevorzugt C₃- und C₄-Alkyl wie n-Propyl, iso-Propyl, n-Butyl, iso-Butyl, tert.-Butyl; C₃-C₈-Cycloalkyl, bevorzugt Cyclopropyl, Cyclobutyl, Cyclopentyl, Cyclohexyl; C₂-C₆-Alkenyl, bevorzugt Vinyl und Propenyl; C₃-C₈-Cycloalkenyl, bevorzugt Cyclopentenyl und Cyclohexenyl; Aryl oder substituiertes Aryl, bevorzugt Phenyl, mono- oder disubstituiertes Phenyl, Naphtyl oder Heteroaryl wie Furyl, Thienyl oder Pyridyl.

Der Rest R³ besitzt die folgende Bedeutung:

H; C₁-C₆-Alkyl, bevorzugt C₁-C₄-Alkyl wie Methyl, Ethyl, n-Propyl, iso-Propyl, n-Butyl, iso-Butyl, tert.-Butyl; C₃-C₈-Cycloalkyl, bevorzugt Cyclopropyl, Cyclobutyl, Cyclopentyl, Cyclohexyl; Aryl oder substituiertes Aryl, bevorzugt Phenyl, mono- oder disubstituiertes Phenyl; C₁-C₆-Alkoxy, bevorzugt Methoxy, Ethoxy, n-Propoxy oder iso-Propoxy; C₁-C₈-Acyl, bevorzugt Formyl, Acetyl, Propionyl, Pivaloyl, n-Butyryl, iso-Butyryl, Benzoyl.

Der Rest Ar besitzt folgende Bedeutung:

Aryl, bevorzugt Phenyl, Naphtyl; Heteroaryl, bevorzugt Furyl, Thienyl, Pyrryl oder Pyridyl; substituiertes Aryl, bevorzugt mono oder disubstituiertes Phenyl; substituiertes Heteroaryl, bevorzugt mono oder disubstituiertes Furyl, Thienyl, Pyrryl oder Pyridyl, wobei die Substituenten gleich oder verschieden sein können und folgende Bedeutung haben: Alkyl, Cycloalkyl, Alkoxy, Amino, Alkylamino, Hydroxy, Halogen, Acyl, besonders bevorzugt Acyloxy oder Acylamino sowie Alkoxycarboxy oder Aminocarboxy, Hydroxycarboxy, Oxysulfonyl, Aminosulfonyl.

Die C=C Doppelbindung der erfindungsgemäßen Arylaminomethylencampherderivate (1) liegt nach der Synthese sowohl in der E- als auch in der Z-Konfiguration vor. Durch Kristallisation erhält man bevorzugt Verbindungen der Formel (1), in der die Substituenten an der C=C Doppelbindung in der trans-Position vorliegen.

Die erfindungsgemäßen Verbindungen (1) zeichnen sich durch eine hohe Photostabilität aus und sind als Lichtschutzmittel, vor allem als UV-A Filter, insbesondere für kosmetische Anwendungen besonders geeignet. Die UV-Filterwirkung kann aber auch zur Stabilisierung von Kunststoffen, Farbstofformulierungen oder Lacken ausgenutzt werden.

Kosmetische Präparate oder Zubereitungen enthalten die Verbindungen (1) im allgemeinen zu 0,1 bis 15 Gew.-%, vorzugsweise zu 5-10 Gew.%, bezogen auf die Formulierung, neben den in der Kosmetik üblichen Trägerstoffen oder Verdünnungsmitteln und gegebenenfalls üblichen kosmetischen Hilfsstoffen.

Von der Art des Trägers , Hilfsstoffes oder Verdünnungsmittel hängt es ab, ob das fertige lichtschutzmittelhaltige Präparat eine Lösung, ein Öl, eine Creme, eine Salbe, eine Lotion. ein Gel oder ein Pulver ist. Derartige Zubereitungen können beispielsweise der Zeitschrift "Seifen, Öle, Fette, Wachse", 1955, Seite 147 entnommen werden.

Üblicherweise verwendete kosmetische Hilfsstoffe, die als Zusätze in Betracht kommen, sind beispielsweise Emulgatoren, wie Fettalkoholethoxylate, Sorbitanfettsäureester oder Lanolinderivate, Verdickungsmittel, wie Carboxymethylcellulose oder vernetzte Polyacrylsäure, Konservierungsmittel und Parfüms.

Grundlage für Sonnenschutzöle sind beispielsweise pflanzliche Öle, wie Erdnußöl, Olivenöl, Sesamöl, Baumwollsamenöl, Kokosöl, Traubenkernöl, Ricinusöl oder Mineralöle, wie Vaselineöl, oder insbesondere flüssiges Paraffin, synthetische Fettsäureester und Glyceride. Grundlage für Salben sind beispielsweise Vaseline, Lanolin, Eucerin oder Polyethylenglykole.

Grundlagen für Cremes sind beispielsweise fettreiche Cremes, Glycerin-, Polysaccharid-, Tylosecreme, für Cremes auf Basis von Fetten und Wachsen Cetylalkohol, Lanolincreme, Kakaobutter, Bienenwachs, Stearinsäure, Stearylalkohol, Glycerinmonostearat, native oder mineralische Öle und Fette.

Grundlagen für Emulsionen sind beispielsweise Mischungen aus Stearylglykol, einem pflanzlichen und/oder Mineralöl, wie Mandelöl, Paraffinöl und Vaseline, und Wasser oder Mischungen aus Ethylalkohol, Wasser, Lanolin und Tragant, oder Mischungen aus Ethylalkohol, Stearin, Wasser, Tragant und Glycerin oder Mischungen aus Stearinsäure, Paraffinöl, Propyl- oder Isopropylalkohol und Wasser.

Die erfindungsgemäßen Verbindungen können als einzige UV-Absorber in den entsprechenden Zubereitungen eingesetzt werden; man kann sie jedoch auch in Kombination mit anderen UV-Absorbern, insbesondere UV-B-Absorbern einsetzen.

Beispiele für solche Verbindungen sind p-Aminobenzoesäureethylester, p-Methoxyzimtsäure-2-ethylhexylester, 2-Ethylhexyl-2-cyano-3,3-diphenylacrylat, 2-Phenylbenzimidazolsulfonsäure und Salze, 2-Hydroxy-4-methoxybenzophenon, 2-Hydroxy-4-methoxybenzophenon-5-sulfonsäure, 3-(4'-Methylbenzyliden)-d,l-campher, 2,4,6-Trianilino-(p-carbo-2'-ethylhexyl-1'-osy)-1,3,5-triazin.

Zur Herstellung der erfindungsgemäßen Verbindungen (1) ist folgende allgemeine Arbeitsvorschrift besonders bevorzugt zu verwenden:

Equimolare Mengen (22 mmol) der Hydroxymethylencampherverbindung (2) und des Amins (3) werden in 20 ml Methanol gelöst, mit 0,1 ml konz. Salzsäure versetzt und für 2 h auf 65°C erhitzt. Nach Abkühlen des Reaktionsgemisches auf 10°C kristallisiert das Wertprodukt (1) aus, das sich durch Filtration, Waschung und anschließender Trocknung analysenrein isolieren läßt.

### Beispiel 1

Gemäß der allgemeinen Arbeitsvorschrift wurden 4 g (22 mmol) Hydroxymethylencampher mit 5,45 g (22 mmol) p-Aminobenzoesäure-2'-ethylhexylester umgesetzt. Man erhielt 9 g einer Verbindung der Formel: 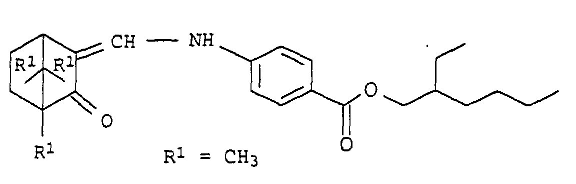
- UV(CH₃OH):: λₘₐₓ = 358 nm, E₁¹ = 1044
- IR:: τ = 3220, 2959, 1711, 1692, 1636, 1602 (s), 1587, 1263 (s), 1176, 1105, 1072 cm⁻¹.
- ¹H-NMR (CDCl₃):: δ = 9,87 (d, 1H, J = 11,2 Hz)
7,95 (d, 2H, J = 7,6 Hz),
6,91 (m, 3H),
4,16 (d, 2H, J = 8,2 Hz),
2,40 (m, 1H),
2,01 (m, 1H),
1,68 (m, 2H),
1,32 (m, 10H),
0,96 (m, 15H).

### Beispiel 2

Gemäß der allgemeinen Arbeitsvorschrift wurden 4 g (22 mmol) Hydroxymethylencampher mit 4,29 g (22 mmol) o-Aminobenzoesäurebutylester umgesetzt. Man erhielt 4,9 g einer Verbindung der Formel: 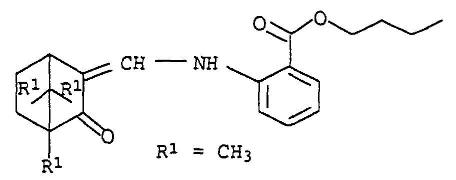
- UV(CH₃OH) :: λₘₐₓ = 358 nm, E₁¹ = 802
- IR:: τ = 3274, 2943, 1687, 1622 (s), 1602, 1592, 1272, 1246, 1156 cm⁻¹.
- ¹H-NMR (CDCl₃):: δ = 10,13 (d, 1H, J = 16,2 Hz),
7,98 (d, 1H, J = 6,9 Hz),
7,68 (d, 1H, J = 16,2 Hz),
7,49 (t, 1H, J = 7,2 Hz),
7,21 (d, 1H, J = 6,9 Hz),
6,87 (d, 1H, J = 7,2 Hz),
4,37 (t, 2H, J = 8,1 Hz),
2,81 (m, 1H),
2,11 (m, 1H),
1,4-1,6 (m, 9H),
0,99 (m, 10H).

### Beispiel 3

Analog zu den Beispielen 1 und 2 wurden die in Tab. 1 aufgeführten Verbindungen 3.1 bis 3.15 hergestellt

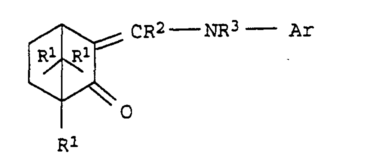

**Tabelle 1**

| Verbind. | R¹ | R² | R³ | Ar |
|---|---|---|---|---|
| 3.1 | CH₃ | H | H | 4-CH₃O-Ph |
| 3.2 | CH₃ | H | H | 2,4-Di-CH₃O-Ph |
| 3.3 | CH₃ | CH₃ | H | 4-CH₃O-Ph |
| 3.4 | CH₃ | CH₃ | CH₃ | 4-CH₃O-Ph |
| 3.5 | H | H | H | 4-CH₃O-Ph |
| 3.6 | CH₃ | H | H | 2-(H₂N-CO)-Ph |
| 3.7 | CH₃ | H | H | 4-(H₂N-CO)-Ph |
| 3.8 | H | H | H | 2-(CH₃O-CO)-Ph |
| 3.9 | H | H | H | 4-(C₈H₁₇O-CO)-Ph |
| 3.10 | CH₃ | H | H | 4-Chinolin |
| 3.11 | CH₃ | H | H | 2-CO₂H-Ph |
| 3.12 | CH₃ | H | H | 4-SO₃H-Ph |
| 3.13 | CH₃ | H | H | 4-SO₂NH₂-Ph |
| 3.14 | CH₃ | CH₃ | H | 2-CO₂H-Ph |
| 3.15 | CH₃ | CH₃ | H | 2-(nC₄H₉O-CO)-Ph |

## Patentansprüche

1. Verwendung von Arylaminomethylencampherderivaten der Formel (1) 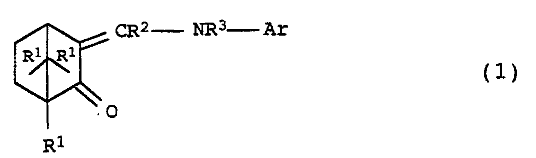 wobei die C=C Doppelbindung in der Z oder E Konfiguration vorliegt und die Variablen folgende Bedeutung haben:
R¹ H, CH₃,
R² H, C₁-C₆-Alkyl, C₃-C₈-Cycloalkyl, C₂-C₆-Alkenyl, C₃-C₈-Cycloalkenyl, Aryl oder substituiertes Aryl,
R³ H, C₁-C₆-Alkyl, C₃-C₈-Cycloalkyl, Aryl, substituiertes Aryl, C₁-C₆-Alkoxy, C₁-C₈-Acyl,
Ar Aryl, substituiertes Aryl, Heteroaryl oder substituiertes Heteroaryl
als. Lichtschutzmittel in kosmetischen Zubereitungen.

2. Verwendung nach Anspruch 1, **dadurch gekennzeichnet, daß** die C=C Doppelbindung in der E Konfiguration vorliegt.

3. Verwendung nach Anspruch 1, **dadurch gekennzeichnet, daß** R¹ = CH₃, R² = R³ = H und Ar = 2-(Alkoxycarboxy)-Phenyl bedeuten.

4. Arylaminomethylencampherderivate der Formel (1) 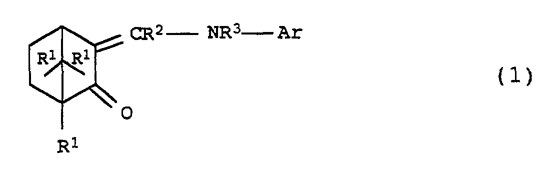 wobei die C=C Doppelbindung in der Z oder E Konfiguration vorliegt und die Variablen folgende Bedeutung haben:
R¹ CH₃;
R² und R³ Wasserstoff;
Ar 2-(Alkoxycarboxy)-Phenyl.

5. Arylaminomethylencampherderivate der Formel (1) 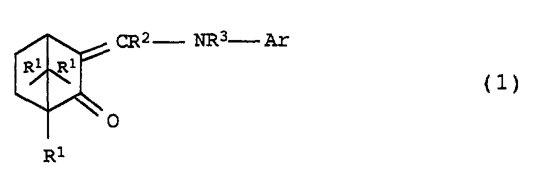 wobei die C=C Doppelbindung in der Z oder E Konfiguration vorliegt und die Variablen folgende Bedeutung haben:
R¹ CH₃;
R² und R³ Wasserstoff;
Ar

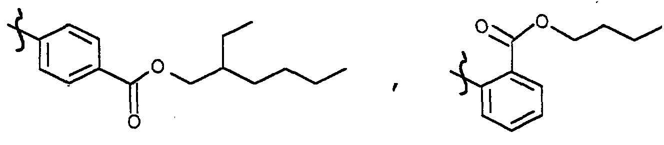

6. Arylaminomethylencampherderivate der Formel (1) 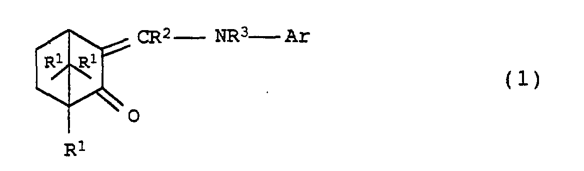 wobei die C=C Doppelbindung in der Z oder E Konfiguration vorliegt und die Variablen folgende Bedeutung haben:
R¹ bis R³ Wasserstoff;
Ar

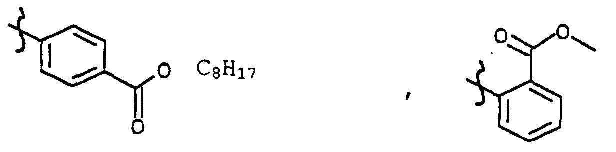

7. Arylaminomethylencampherderivate der Formel (1) 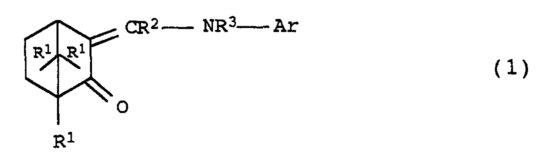 wobei die C=C Doppelbindung in der Z oder E Konfiguration vorliegt und die Variablen folgende Bedeutung haben:
R¹ und R² CH₃;
R³ Wasserstoff;
Ar

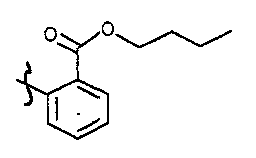

## Claims

1. The use of arylaminomethylenecamphor derivatives of the formula (1) 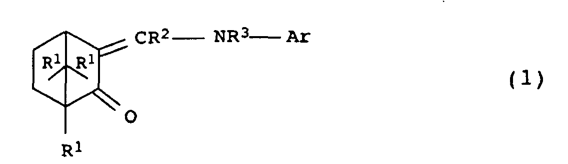 where the C=C double bond is in the Z or E configuration, and the variables have the following meanings:
R¹ H, CH₃,
R² H, C₁-C₆-alkyl, C₃-C₈-cycloalkyl, C₂-C₆-alkenyl, C₃-C₈-cycloalkenyl, aryl or substituted aryl,
R³ H, C₁-C₆-alkyl, C₃-C₈-cycloalkyl, aryl, substituted aryl, C₁-C₆-alkoxy, C₁-C₈-acyl,
Ar aryl, substituted aryl, hetaryl or substituted hetaryl,
as sunscreen agents in cosmetic compositions.

2. The use as claimed in claim 1, wherein the C=C double bond is in the E configuration.

3. The use as claimed in claim 1, wherein R¹ is CH₃, R² = R³ = H and Ar is 2-(alkoxycarboxy)phenyl.

4. An arylaminomethylenecamphor derivative of the formula (1) 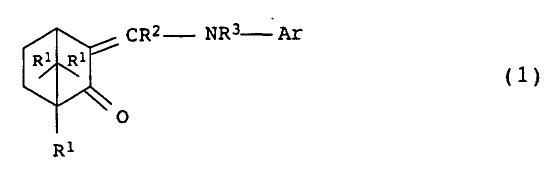 where the C=C double bond is in the Z or E configuration, and the variables have the following meanings:
R¹ CH₃;
R² and R³ hydrogen;
Ar 2-(alkoxycarboxy)phenyl.

5. An arylaminomethylenecamphor derivative of the formula (1) 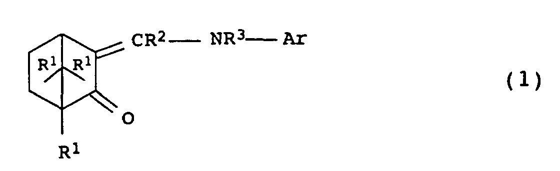 where the C=C double bond is in the Z or E configuration, and the variables have the following meanings:
R¹ CH₃;
R² and R³ hydrogen;
Ar

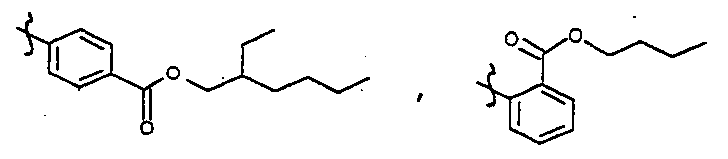

6. An arylaminomethylenecamphor derivative of the formula (1) 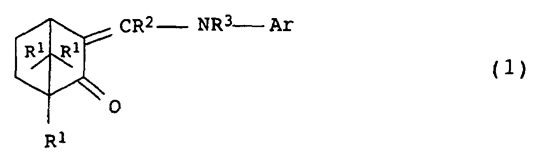 where the C=C double bond is in the Z or E configuration, and the variables have the following meanings:
R¹ to R³ hydrogen;
Ar

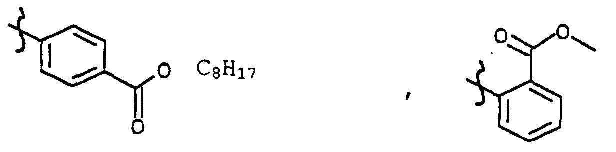

7. An arylaminomethylenecamphor derivative of the formula (1) 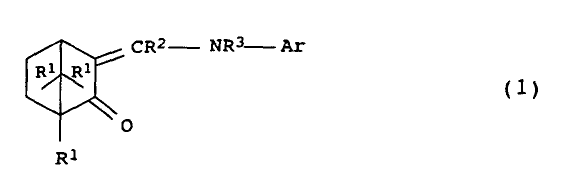 where the C=C double bond is in the Z or E configuration, and the variables have the following meanings:
R¹ and R² CH₃;
R³ hydrogen;
Ar

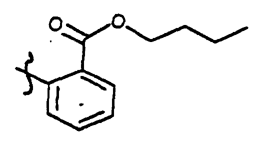

## Revendications

1. Utilisation de dérivés d'arylaminométhylènecamphre de formule (1) 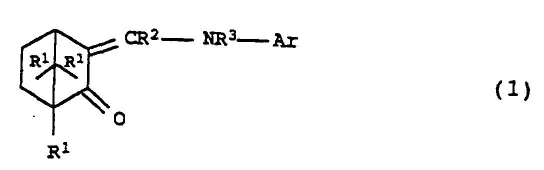 où la double liaison C=C se présente dans la configuration Z ou E et les variables ont la signification suivante:
R¹ H, CH₃,
R² H, alkyle en C₁-C₆, cycloalkyle en C₃-C₈, alcényle en C₂-C₆, cycloalcényle en C₃-C₈, aryle ou aryle substitué,
R³ H, alkyle en C₁-C₆, cycloalkyle en C₃-C₈, aryle, aryle substitué, alcoxy en C₁-C₆, acyle en C₁-C₈,
Ar aryle, aryle substitué, hétéroaryle ou hétéroaryle substitué,
comme agents protecteurs contre la lumière dans des préparations cosmétiques.

2. Utilisation selon la revendication 1, **caractérisée par le fait que** la double liaison C=C se trouve dans la configuration E.

3. Utilisation selon la revendication 1, **caractérisée par le fait que** R¹ = CH₃, R² = R³ = H et Ar = 2-(alcoxycarboxy))-phényle.

4. Dérivés d'arylaminométhylènecamphre de formule (1) 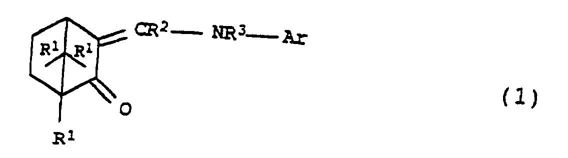 où la double liaison C=C se trouve dans la configuration Z ou E et les variables ont la signification suivante:
R¹ CH₃;
R² et R³ hydrogène;
Ar 2-(alcoxycarboxy)-phényle.

5. Dérivés d'arylaminométhylènecamphre de formule (1) 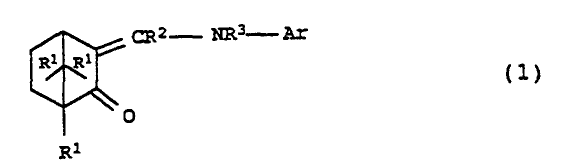 où la double liaison C=C se trouve dans la configuration Z ou E et les variables ont la signification suivante:
R¹ CH₃;
R² et R³ hydrogène;
Ar

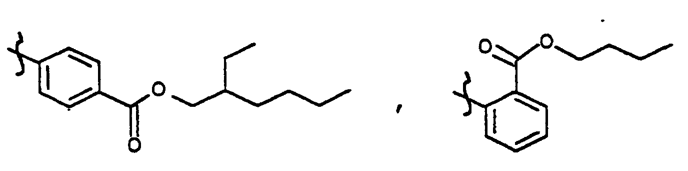

6. Dérivés d'arylaminométhylènecamphre de formule (1) 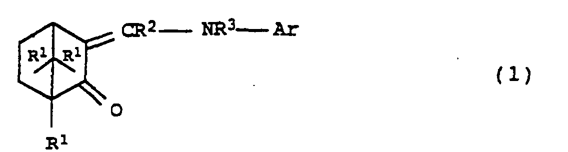 où la double liaison C=C se trouve dans la configuration Z ou E et les variables ont la signification suivante:
R¹ à R³ hydrogène;
Ar

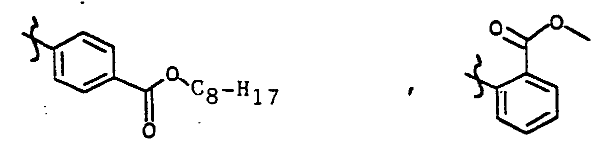

7. Dérivés d'arylaminométhylènecamphre de formule (1) 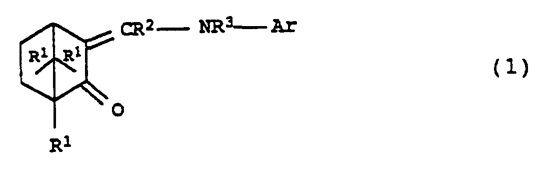 où la double liaison C=C se trouve dans la configuration Z ou E et les variables ont la signification suivante:
R¹ et R² CH₃;
R³ hydrogène;
Ar
